# EUROPEAN PATENT SPECIFICATION

(11) **EP 4 382 600 B1**
(45) Date of publication and mention of the grant of the patent: **04.03.2026**
(21) Application number: 21940505.7
(22) Date of filing: 10.11.2021
(51) Int. Cl.: C12N 15/11, C12N 15/10, C12Q 1/6869

(54) **DOUBLE-LAYER MICROSPHERE WITH OLIGONUCLEOTIDE SEQUENCE FOR SINGLE-CELL SEQUENCING**
DOPPELSCHICHTIGE MIKROKUGEL MIT OLIGONUKLEOTIDSEQUENZ ZUR EINZELZELLSEQUENZIERUNG
MICROSPHÈRE À DOUBLE COUCHE AVEC SÉQUENCE OLIGONUCLÉOTIDIQUE POUR LE SÉQUENÇAGE MONOCELLULAIRE

(30) Priority: 15.05.2021 CN 202110530676
(43) Date of publication of application: 12.06.2024
(73) Proprietor: Mobidrop (Zhejiang) Co., Ltd., Zhejiang Province, 314501 (CN)
(72) Inventor: ZHENG, Wenshan, Jiaxing, Zhejiang 314501 (CN); PEI, Hao, Jiaxing, Zhejiang 314501 (CN); XU, Yunfei, Jiaxing, Zhejiang 314501 (CN); ZHANG, Xiaojin, Jiaxing, Zhejiang 314501 (CN)
(74) Representative: Bandpay & Greuter
(86) International application number: PCT/CN2021/129701
(87) International publication number: WO 2022/242061

(56) References cited:
- WO-A1-2020/134612
- CN-A- 1 290 721
- CN-A- 102 861 541
- CN-A- 110 054 696
- CN-A- 112 251 504
- CN-A- 113 234 718
- US-A1- 2018 088 112
- US-A1- 2019 381 497

## Description

The present application claims the priority of Chinese patent application 2021105306761 with the filing date of 2021/5/15. The present application refers to the entire text of the Chinese patent application described above.

### TECHNICAL FIELD

The present invention belongs to single cell sequencing reagent, and specifically relates to a double-layered microsphere with oligonucleotide sequences.

### BACKGROUND

Single-cell RNA-sequencing (scRNA-seq) is a new technique rising in recent years, and can obtain full transcriptome expression profiles at single cell level, and perform high-throughput sequencing after amplification, thereby efficiently detecting the gene expression levels in a single cell, and having important application value in the fields of scientific research and precision medicine, such as tumor diagnosis and treatment, targeted drug design, stem cell development and differentiation.

The single cell transcriptome sequencing can simultaneously perform sequencing on hundreds of thousands of cells, this is because each cell has a unique barcode, in order to distinguish each mRNA in each cell, each mRNA is also endowed with a unique molecular identifier (UMI). At present, the microfluidic platform for single cell sequencing is mainly characterized in that placing a single cell and a single microsphere with the molecular barcode in a microporous environment, and after the cell lysis, different cells can be labelled by specific barcodes, upon reverse transcription, different mRNAs of the same cell are marked by different molecular barcodes. The method can track the cell source of each gene, and can quantify the mRNA, significantly improves the sequencing throughput, reduces the sequencing cost, and reduces the influence of amplification bias. However, there are the latest single cell sequencing techniques based on droplet microfluidic technology, which can integrate complex multiple steps into a microfluidic chip; however, conventional microspheres with molecular barcodes have many problems such as high manufacturing costs, preparation difficulty, low preparation success rate, error from batch effects, and the like. The most serious challenge is that it is difficult to encapsulate barcoding microspheres in droplets at high singlet rate (i. e. one droplet has one barcoding microsphere inside) , which affects the accuracy and reproducibility of single cell sequencing results, and at the same time, this is also one of the reasons that the single cell sequencing cost is expensive and cannot be widely applied.

### SUMMARY OF THE INVENTION

In view of the above mentioned problems, the present invention provides a double layer microsphere with oligonucleotide sequences, the outer layer of which is made of a soluble hydrogel, and an inner layer thereof is a magnetic microsphere with oligonucleotide sequences, which finally makes the double layer microspheres inherit traditional hard magnetic microspheres' advantage of easy-to-connect with the oligonucleotide sequences, and at the same time, the outer layer large volume microsphere made of soft hydrogel materials makes it easy to achieve high singlet rate (one droplet has one barcoding microsphere inside).

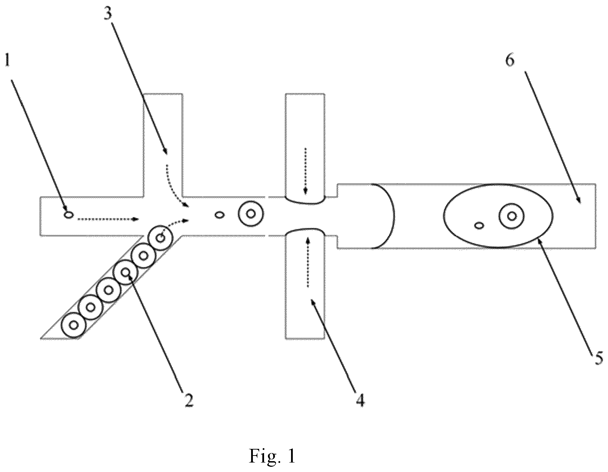

The present invention discloses a double layer microsphere with oligonucleotide sequences, and also discloses a method for preparing such double layer microsphere with oligonucleotide sequences. The schematic diagram of sequencing process using the double layer microsphere provided in the present invention is shown in Fig 1.

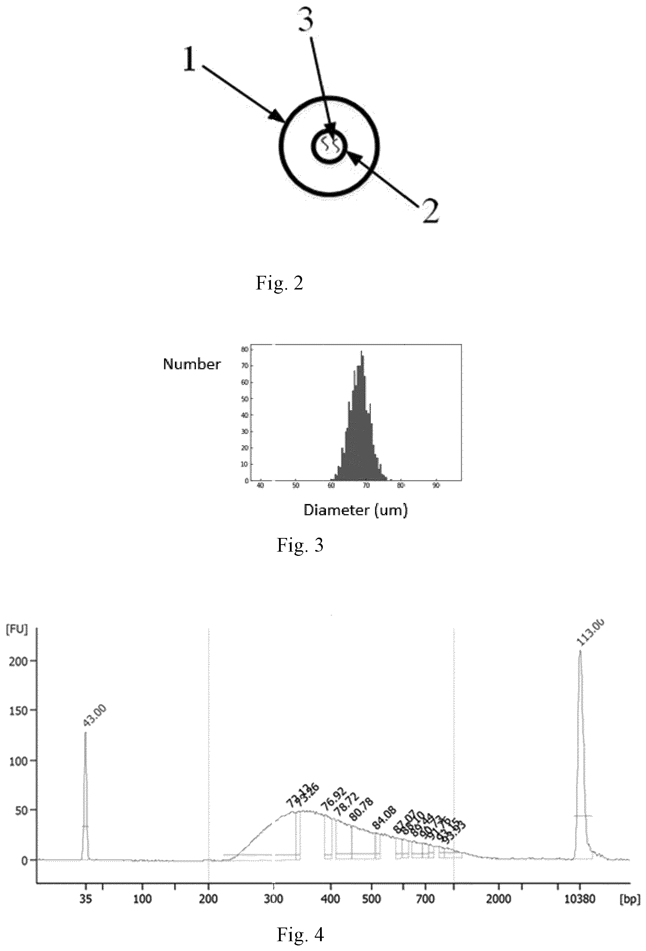

The double layer microsphere with oligonucleotide sequences of the present invention, the outer layer is made of a soluble hydrogel, and the inner layer thereof is a magnetic microsphere with oligonucleotide sequences; structural schematic diagram of the present invention is shown in Fig 2.

The oligonucleotide sequence has at least two parts, C region and D region; the C region is a universal DNA sequence for sequencing library construction; and the D region sequence length is 10bp-200bp, and is used for identifying nucleic acids in different single cells, and includes a sequence for combining and capturing the nucleic acids in cells via complementary base pairing.

Further, the selection of the D region sequence includes at least three parts, D1, D2 and D3, wherein the sequence of D1 (SEQ ID NO: 1) is ACACTCTTTCCCTACACGACGCTCTTCCGATCT[X]A, the sequence of D2 is CTG [Y] A, and the sequence of D3 is CTG [Z] [polyT]; wherein each X, Y, or Z represents any DNA sequence having a length of 4-14 bp (where X is represented by "N" in the sequence table), and polyT represents 10-35 T.

Further, the oligonucleotide sequences of the C region and the D region have three segments, and the three segments are sequentially connected to the magnetic microspheres in sequence by means of chemical bonds and DNA ligase or polymerase.

Further, the microsphere has a double layer structure, the outer layer is made of a soluble hydrogel, which reduces the difficulty of encapsulating barcoding microspheres in droplets at a high singlet rate (capture one microsphere in one droplet) ;

Further, the outer layer material of the microsphere may be selected from polyacrylamide containing a disulfide bond, the disulfide bond-containing polyacrylamide microsphere is soft, resilient, tough, and slightly elastic, and can be dissolved, and is less likely to be blocked in the flow channel than the microspheres made by hard materials;

Further, the diameter of the microspheres is 30-300 microns, it is easy to occupy the flow channel, so that the droplets can be arranged in a single line manner in the flow channel;

Further, the double layer microspheres each can independently occupy one flow channel and the double layer microspheres are arranged in order in actual use, so that the time intervals of the microspheres entering the subsequent flow channel are constant, and the subsequent microsphere encapsulation in droplets at high singlet rate or other control can be guaranteed and facilitated;

Further, the double layer microspheres significantly reduce the difficulty of encapsulating barcoding microspheres in droplets at a high singlet rate (one droplet has one barcoding microsphere), and improve the accuracy and reproducibility of single cell sequencing results;

Further, the inner magnetic microspheres of the double layer microspheres are the most commonly used commercially-available microspheres, which can be modified and connected with oligonucleotides more easily than other materials.

Further, the surface of the polyacrylamide microsphere is modified and linked with more than two different types of oligonucleotide sequences, so that the types of microspheres are numerous.

At the same time, the present invention provides a method for preparing a double layer microsphere with oligonucleotide sequences, including:
(1) synthesizing three oligonucleotide sequences C+D1, D2, D3 and corresponding auxiliary sequences RC2, RC3 respectively, where RC2 is a complementary sequence of ACTG [Y] ACTG, RC3 is a complementary sequence of [Z] [PolyT];
(2) adding the oligonucleotide sequence C+D1 into a microporous plate, adding the magnetic microspheres to connect with C+D1 to obtain a magnetic microsphere mixture A;
(3) adding DNA ligase into the magnetic microsphere mixture A obtained in step (2) to obtain a mixed system M; adding the oligonucleotide sequence D2 into a new microporous plate, adding the auxiliary sequence RC2 to obtain the microporous plate containing D2; adding the mixed system M into the microporous plate containing D2 to obtain a polyacrylamide microsphere mixture B connected to D1 and D2;
(4) adding DNA ligase into the magnetic microsphere mixture B obtained in step (3) to obtain a mixed system N, adding the oligonucleotide sequence D3 into a new microporous plate, adding the auxiliary sequence RC3 to obtain the microporous plate containing D3; adding the mixed system N into the microporous plate containing D3 to obtain the magnetic microspheres connected to D1, D2 and D3.
(5) by using droplet microfluidic method, coating the magnetic microsphere having a molecular barcode sequence generated in (4) into a polyacrylamide outlayer to form a double layer microsphere.

The double layer microsphere with the oligonucleotide sequences of the present invention has the following beneficial effects:
1. The double layer microsphere of the present invention has a large volume, which can be customized according to the flow channel size of a common microfluidic chip that often used on the market; 2. the texture of the polyacrylamide microspheres is slightly soft and elastic, so that the polyacrylamide microspheres can independently occupy one flow channel and are arranged in order in actual use, this obviously reduces the difficulty for achieving high singlet rate, i. e. one droplet captures one double-layer microsphere; 3. the inner layer small magnetic microspheres are the most commonly used commercially-available microspheres, which can be easily connected to the oligonucleotides than other materials, so that the double layer microspheres inherit the advantage of the traditional hard magnetic microspheres that can be easily modified with surface oligonucleotide sequences; 4. the difficulty of the achieving high singlet rate (the microsphere-containing droplets have only one microsphere in each droplet) is reduced, which is attributed to the large-volume of the outer layer microspheres, and the accuracy and reproducibility of the single cell sequencing results are improved as well.

### DESCRIPTION OF THE DRAWINGS

FIG. 1 is a schematic diagram of single cell sequencing process using double layer microsphere barcoding microspheres, including target cell 1 for single cell sequencing, double layer microsphere 2 with oligonucleotide sequences, mixed solution 3 containing cell lysis solution, oil 4 with surfactant, droplet 5 that successfully encapsulates a target cell and a double layer microsphere with oligonucleotide sequences, and droplet collection outlet 6.
FIG. 2 is a structural schematic diagram of the present invention, the microsphere includes double layers, the outer layer is a hydrogel material (polyacrylamide) 1, and the inner layer is a hard microsphere 2 and oligonucleotide sequences 3.
FIG. 3 is a diameter distribution diagram of the double layer microsphere.
FIG. 4 is a sample DNA library obtained by traditional Drop-seq microbeads.
FIG. 5 is a DNA library obtained by the double layer microspheres as-prepared in an embodiment of the present invention.

### DETAILED DESCRIPTION OF THE INVENTION

In order to make the objectives, technical solutions, and advantages of the present invention clearer, the present invention will be further described in detail below with reference to the accompanying drawings and embodiments. It should be understood that the specific embodiments described here are only used to explain the present invention, and are not intended to limit the present invention.

A person skilled in the art may appropriately improve process parameter implementation by referring to the content of this article. It is particularly noted that all similar alternatives and modifications will be apparent to those skilled in the art, and are considered to be included in the present invention. The method and application of the present invention have been described by the preferred embodiments, and the relevant personnel can obviously make changes or appropriate changes and combinations to the methods and applications described herein without departing from the contents, spirit and scope of the present invention, so as to implement and apply the technology of the present invention. The methods, apparatuses, and materials in the following embodiments are all conventional methods, devices, and materials in the art if not specifically described, and are commercially available.

### Embodiment: preparation of double layer polyacrylamide microsphere

Materials and reagents: 2-Morpholine ethanesulfonic acid (MES), 1-(3-dimethylaminopropyl)-3-ethyl carbodiimide hydrochloride (EDC), Tween-20 are purchased at Sigma-Aldrich; T4 ligase and 10× Ligation buffer are both purchased at New England Biolabs (NEB); magnetic beads are purchased at SUZHOU VDO Biotech Co., Ltd.; DNase/RNase-Free Water (nuclease-free water) is purchased at Thermo Fisher Scientific; TE (TE buffer) is purchased at Sangon Biotech (Shanghai) Co., Ltd.; 40% acrylamide solution, ammonium persulfate, N, N, N', N'-tetramethyl ethylenediamine are purchased at Sigma-Aldrich; 1M Tris-HCI (pH value 8.0), 5M NaCl, N, N' bis (acryloyl) cystamine are purchased at Thermo Fisher Scientific; Droplet Generation Oil for Probes is purchased at BioRad; HFE 7100 is purchased at 3M.

### 1, Preparation of oligonucleotide:

the sequence of D1 (SEQ ID NO: 1) is ACACTCTTTCCCTACACGACGCTCTTCCGATCT [X] A;
the sequence of D2 is: CTG [Y] A;
the sequence of D3 is: CTG [Z] [polyT], where polyT represents 20 T;
RC2 is a complementary sequence of ACTG [Y] ACTG;
RC 3 is a complementary sequence of [Z] [PolyT], where polyT represents 20 T;
X, Y, and Z sequences described above each represents any DNA sequence having a length of 4-14 bp. In order to facilitate the experiment, in a specific embodiment, 96 sequences are selected for description, and the specific sequences of the 96 sequences are as follows:

| **X sequence** | **Y sequence** | **Z sequence** | **X sequence** | **Y sequence** | **Z sequence** |
|---|---|---|---|---|---|
| ATCGATCG | AGGTTCTC | TGAATGAC | AATGGCCG | TACAGATA | TCGATGAC |
| ACTGACTG | ATTCCTAA | ATGTTTTA | AAGAAAAG | TTAGCTGA | TCCTGGGA |
| GGTTCTGT | GACATCCC | TCCGGCAT | TAACGCGC | AGATTATA | GGAGAAGG |
| TCGCTGCT | CGACACTC | CCGAAGGG | CCGGGCGA | CCATCGTA | CTTTACAG |
| GGGGTCGC | CCAGACCA | TCTATGGG | CCGTGTCG | AGGGTCAG | TCTACATT |
| TATCGGTG | CTAGCTCA | GCCCAGCC | GCAACTAG | TGTAGCTA | TTCATTTT |
| TGCACTGC | ATGCAGGG | ACGGTCGG | GTTGACAT | TACAAGCA | TAACGCAA |
| ATGATGAC | CCACGGTC | GTAGGGTT | GAATTGCC | GCGTGCAA | ATCCCCTC |
| AGGTATCG | CGAGCTTA | CGCAAGCG | AGTCTATA | TAAAGTGA | CTGCGTTG |
| ACCCGCGC | ACCTGTTG | TAAGACTT | ACCACTTG | CAGATGGA | TCCCAATG |
| CTCTCTAA | TCCGGTAA | CAGTATGT | TGCTCTAC | TATGTAAC | ATTGGACA |
| CCTAGGGG | CGTTCCTC | CGAGAGCA | CGAGGGGC | CATCGTTT | CCGCGGAA |
| GGCTCTGG | TTCCAACA | TCTGATCT | GCGAGTGT | ATCTCGGG | CCGATTGT |
| GCGCTCGG | AACAAGTG | GTAACCAG | GAAGTGGC | TACTAATG | GCGCGATC |
| GACTCGAC | ATTATGCT | TTCATCGG | CAAGGGCT | GAGACATG | GCGCCTTT |
| AAGTGAAC | TTCCCTAG | GATATTTT | GCATCAGG | TGCCATTC | AGCGGGAT |
| ACGCCACC | TTAAGCCA | GAGCTCAT | GAGAACGA | GTCGTCGC | GTCCCCAT |
| CTTGAAGA | TGGAAGAG | ACCGCTCG | ATACGAAT | GAGACTCT | ATCTTCAG |
| AAGGATAT | CAAGCCTA | TCCGCGTC | GAGTTTGG | TCTCTACA | CACACCTT |
| ACTACAAG | CTGCAGAA | GGTCGAGT | CAGACAGG | TTACCGCC | CGGTGAAG |
| ATCGGACG | TAATCTTT | CGACTACG | GTAGTTAA | CTAGGTGG | GCCTGGTA |
| ACGTTCTA | TTGCGGCT | CTTTCTAG | AAATCCAT | TCTCGGCT | CCGGAGGC |
| TTCCTTGT | CCGTCACT | GAATCAGC | CATTGATT | GGTGCGGG | CACTAGGG |
| CACTATAG | GGACTGGC | GCACGTCT | CTATTGGT | CAACCGCC | CGATGCAA |
| TTTGCCCT | CCTGGTCA | TGTTATCA | CCGTTGTA | CACGACCT | ATTCTAGC |
| TCAATACT | GCGTCTGC | GTATGGGA | TGATCTAA | GAGGTATC | TGCGGGCA |
| GCTTCTCA | GAAAAGGT | GCAATTTG | CCATGCCA | TTGGCGAG | AACAATAT |
| TTACTCGG | GGGCACTG | ACGCAAGT | CTCGAGTG | GAGGGCCA | AATGACCC |
| TTTGGGAG | AACAACGC | GGCCGGAA | AGATCCTA | CTAATGCG | TGGAGCGT |
| CACGTCTA | GTTTGCGC | AGGCTTTT | TTGGCAGT | GACTCTTA | TGCGAATT |
| CAGCAACG | CGCTGCTC | TCGAAAAG | TCAGATTC | GTCTACAG | CTGCTACC |
| TGAGTATT | GAACGCCC | CATTGGAC | CCCTCTGG | GAAATGCT | ACACCAAA |
| GGACCTGT | TCAGTGGA | CCCGGAAC | TTCACTCA | AAAGAGGA | CTGCCAAG |
| AACCGGGA | TCTCGCGA | CGCGGCGT | ATCACATG | CAGCGTCG | AAACCTTC |
| CCCCGCTC | GGCGTGGC | TTTCACGT | ACAGCCCA | CGTAAAGT | TGCCATGA |
| ACAATGCT | AAATGTGC | AAATGAGG | ACGGCCAG | ATGCTACA | CGTCAAAT |
| CAAGTTCT | TGATGACT | AAGTGAGA | TATTATGT | TTGAGCTG | CGTTCCCG |
| TAAACATT | ATCACTAA | CGGTGTTT | TCCAGTGT | TTAGTCTC | AATCCAAG |
| TAGTGGTT | ACAGGGTG | TCGCGACT | GTAAGCAT | TGTTTGGG | AATCCCAC |
| CTTCGGCT | GGTCCTTA | AGAAACCC | AAAACACC | TTTTAAGT | CTATTCCG |
| GCAACAAT | AATCGAGA | CGGGAGAC | CCTGTTCC | ATTCGGCC | TAACTAGA |
| CTTCCTTG | CCCGTCAC | GCTTGGCC | GGGGCTCA | CCTTCATG | GCGGTTAT |
| TGTTAGCG | GATCTTGG | GAGAATCC | TAAACAGC | AGGCAAAT | TCCGACCC |
| TCATAAGA | AGCACCAG | TAGGCGGA | GTGTTAGG | AGTAATAA | CCGCCAAC |
| AATCAGGA | GACTATAT | GGCTCAGT | TTTAATAC | AAAGTGTT | GACAGGCA |
| TCGTGTTT | CTCCGTGA | GCCCAAAG | AACCTGTT | GCTCCGCC | GATCTATA |
| CACATGGG | TTTGCGGA | AATGGTGA | CAGATCAG | CAGCTCCC | GCAGATGG |
| TATGGAAT | AGTTTTTC | TTCAGTCT | GTAAAGTG | TCCACAAG | TTCACGTT |

The oligonucleotide sequence used is in a form of solution dissolved in TE buffer with a final concentration of 100 µM.

D2 and RC2, D3 and RC3 should be annealed into double-stranded chains before subsequent ligation reaction.

Mixing 100 µM of D2 and 100 µM of RC2 in equal volumes, the mixed system is heated to 94°C and maintained for 2 min, then slowly cooled. After cooling, a D2-RC2 solution with a concentration of 50 µM is obtained and stored at -20°C (long term) or 4°C (for frequent use in a short time).

Mixing 100 µM of D3 and 100 µM of RC3 in equal volumes, the mixed system is heated to 94°C and maintained for 2 min, then slowly cooled. After cooling, a D3-RC3 solution with a concentration of 50 µM is obtained and stored at -20°C (long term) or 4°C (for frequent use in a short time).

### 2. Preparation of the double layer polyacrylamide microspheres

### (1) First step connection reaction

5 µL of 100 µM D1 is taken and divided into a 96-well plate, each well corresponds to a specific D1 sequence.

About 5 million magnetic beads were taken and washed twice with cleaning solution (0.01 M MES and 0.1% (v/v) Tween-20 aqueous solution). After cleaning, 500 µL of 5 mg/mL EDC solution was added to the magnetic beads, oscillation was performed in a shaker at 37°C for 30 minutes. The EDC solution is removed, 1 mL of 0.01 M MES solution is added, the magnetic beads are suspended and sub-packaged into the 96-well plate of the previous step, 10 µl is added to each well. The 96-well plate is sealed with a sealing film, stored in a 37°C shaker overnight (longer than 8 hours). After the reaction, the magnetic beads in all the wells are collected in a 1.5 mL centrifuge tube, the magnetic beads are washed with 1 mL of 0.01 M MES solution, 1 mL of TE buffer solution, 1 mL of pure water successively. Finally, the magnetic beads are suspended in 1 mL of water, which can be used for the next reaction.

### (2) Second step connection reaction

In each of the wells in the 96-well plate, 10 µl of the product produced in step (1) is added. Then, 20 µL of 50 µM D2-RC2 solution is added, each well corresponds to one specific D2-RC2 sequence. Then 2.5 µL of T4 DNA ligase, 5 µL of T4 DNA ligase Buffer (10X) is added to each well, 12.5 µL of water is added, the mixture is mixed uniformly and then placed in PCR instrument for reaction, the program is maintained at 25°C for 20 min, on ice for 1h, and maintained at 4°C, then the second step connection reaction is completed. The reaction products in the 96-well plate are combined, the magnetic microspheres connected with sequence D2 and sequence D1 are obtained after five times of cleaning by the TE buffer solution, and can be used for the next reaction.

### (3) Third step connection reaction

In each of the wells in the 96-well plate, 10 µL of the product produced in step (2) is added. Then, 20 µL of 50 µM D3-RC3 solution is added, each well corresponds to one specific D3-RC3 sequence. Then 2.5 µL of T4 DNA ligase, 5 µL of T4 DNA ligase Buffer (10X) is added into each well, 12.5 µL of water is added, the mixture is mixed uniformly and then placed in PCR instrument for reaction, the procedure is 105°C for the heated lid, maintain at 25°C for 20 min, maintain on ice for 1 h, and maintain at 4°C to complete the third step connection reaction. The magnetic microspheres connected to sequences D3, D2 and D1 are obtained after the reaction products being cleaned five times by using the TE buffer solution. The magnetic microspheres were suspended in 420 µL of water for the next reaction.

### (4) Preparation of the double layer microspheres

2 µL of 1M Tris-HCI (pH value is 8.0), 2 µL of 5M NaCl, 45 µL of 40% acrylamide aqueous solution, 31 µL (4.26% v/w) of N, N' bis (acryloyl) cystamine aqueous solution, and 10 mg of ammonium persulfate are added to the product in step (3), the mixed solution is used as an aqueous phase of the microfluidic reaction. An oil-phase solution was formulated, by using 2 µL of N, N, N', N'-tetramethylethylenediamine dissolved in 2 mL of Droplet Generation Oil for Probes. The droplet is generated by using the microfluidic chip, the droplet diameter is controlled to be about 60 µm, place the as-prepared droplets at 65°C for at least 8 hours, that is, the encapsulation step is completed. The reaction product is cleaned by HFE 7100 for five times, only the upper aqueous phase is retained during each cleaning. Then the product is further cleaned by TE buffer solution for five times, the lower layer gel-like product is retained during each cleaning. The final product is the double layer microspheres having oligonucleotide sequences on the surface.

The density of the inner magnetic beads and the density of the outer gel in the double layer microspheres are different; the double layer microspheres containing only one inner magnetic bead are separated by high-speed centrifugation method.

The double layer microspheres with the oligonucleotide sequences (hereafter referred to as microspheres) in the present embodiment are arranged in the flow channel in a single row, so that the singlet rate of microsphere encapsulation can be improved via controlling the flow rate. The calculation manner of the singlet rate (i. e., the proportion of the droplets containing only one microsphere in all droplets) is: taking a microscopic picture of droplets, then calculate the number of droplets containing only one microsphere and the total number of all droplets, the ratio of the former to the latter is the singlet rate, that is, the singlet rate = the number of the droplets with one microsphere encapsulated inside / the total number of droplets.

In the present embodiment, by adjusting and matching the flow rate, the singlet rate of the microspheres could reach 80% or higher. This microsphere singlet rate is much higher than those of traditional droplet microfluidic technologies using the magnetic bead microsphere methods (such as Drop-seq), which are lower than 20%.

The particle size distribution of the microspheres prepared by the present method is 30-300 µm, which can be adjusted according to actual needs. In the present embodiment, the diameter of the obtained microspheres is centrally distributed between 60 µm and 75 µm, the size coefficient variation (CV) is 5%. FIG. 3 is a distribution diagram of the particle sizes of the microspheres according to the present embodiment, it can be seen that the particle size distribution of the microsphere prepared in the present embodiment is uniform, and the size CV value is small.

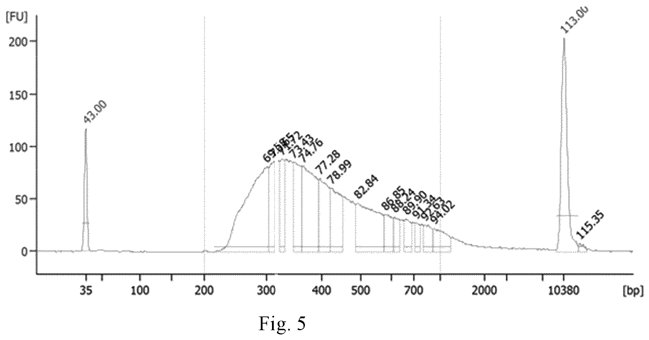

The quality and concentration of the single cell library prepared in the present embodiment are equivalent to or higher than those of the conventional methods based on Drop-seq microbeads. Analyzed by Bioanalyzer 2100 instrument, the concentration of the library obtained by the double layer microspheres in the present embodiment is 12.6 ng/µL (as shown in FIG. 5), while the concentration of the library obtained based on Drop-seq microspheres is 7.3 ng/µL (as shown in FIG. 4).

In the embodiment of the present invention, the polyacrylamide material containing disulfide bond is tough, soft and slightly elastic, and is less likely to block the micro channel than the hard microspheres, and the material can be dissolved.

In the embodiment of the present invention, the target cell sample to be detected is diluted, so that the speed of the target cell entering the main flow channel from the branch flow channel is slower than the speed at which the microspheres entering the main flow channel, thereby realizing high singlet rate.

In the embodiment of the present invention, the size of the droplet is controlled by the flow rate of the oil phase, so that the simultaneous co-encapsulation of target cell and barcoding microsphere into one droplet can be controlled. If the droplet is too small, the probability of the co-encapsulation of the target cell and the barcoding microsphere decreases, if the droplet is too large, the probability of the co-encapsulation of the target cell and the barcoding microsphere also decreases, and the situation that the droplet is difficult to be generated may occur. The double layer soft polyacrylamide microsphere with the oligonucleotide sequences eliminates the difficulty in controlling the speed of the small volume hard microspheres entering the mainstream channel in microfluidic chip, which would cause an increase in the difficulty of singlet co-encapsulation, the polyacrylamide layer containing disulfide bonds is tough, soft, slightly elastic, resilient, making it less prone to block the micrometer-sized channels than the hard microspheres do.

In summary, the double layer microsphere of the present invention has a large volume and is customized according to the size of the flow channel of common microfluidic chips on the market, since the polyacrylamide microsphere containing disulfide bonds is slightly soft in texture, slightly elastic and soluble, it can fully occupy one flow channel and being arranged in order in practical use, the multiplet (one droplet contains more than one barcoding microspheres) rate or the difficulty of the singlet encapsulation can be significantly reduced, so that the microsphere is more suitable for droplet-based single cell sequencing, the accuracy and reproducibility of the single cell sequencing results are significantly improved.

### Sequence table

| | |
|---|---|
| <110> | MobiDrop (Zhejiang) Co., Ltd. |
| < 120 > | A double layer microsphere with oligonucleotide sequence for single cell sequencing |
| <130> | P21408290CF |
| <150> | 2021105306761 |
| <151> | 2021-05-15 |
| <160> | 1 |
| <170> | PatentIn version 3.5 |
| <210> | 1 |
| <211> | 35 |
| <212> | DNA |
| <213> | Artificial Sequence |
| <220> | |
| <223> | D1 |
| <220> | |
| <221> | misc_feature |
| <222> | (34).. (34) |
| < 223 | > N represents any DNA sequence having a length of 4-14 bp, and preferably any DNA sequence having a length of 6 -12 bp |
| <400> | 1 acactctttc cctacacgac gctcttccga tctna |

## Claims

1. A double layer microsphere with oligonucleotide sequences, **characterized in that** an outer layer of the double layer microsphere is made of a soluble hydrogel, and the inner layer thereof is a magnetic microsphere with oligonucleotide sequences;
the oligonucleotide sequence has at least two parts, C region and D region; the C region is a universal DNA sequence for sequencing library construction; and the D region sequence length is 10bp-200bp, and is used for identifying nucleic acids in different single cells, and includes a sequence for combining and capturing nucleic acid in cells.

2. The double layer microsphere according to claim 1, **characterized in that** the oligonucleotide sequences are connected to the inner-layer magnetic microspheres in sequence through chemical bonds created by a method of enzymatic connection.

3. The double layer microsphere according to claim 1, **characterized in that** the diameter of the outer layer of the microsphere is 30-300 microns.

4. The double layer microsphere according to claim 1, **characterized in that** the D region sequence of an inner magnetic microsphere includes at least three parts, D1, D2 and D3, wherein the sequence of D1 is ACACTCTTTCCCTACACGACGCTCTTCCGATCT [X] A, the sequence of D2 is CTG [Y] A, and the sequence of D3 is CTG [Z] [polyT]; wherein X, Y, Z each represents a DNA sequence having a length of 4-14 bp respectively, and polyT represents 10-35 T.

5. The double layer microsphere according to claim 1, **characterized in that** the outer layer is made of polyacrylamide containing disulfide bonds.

6. The double layer microsphere according to claim 1, **characterized in that** a structure of the microsphere is a multi-microporous solid sphere structure, and is soft, tough and slightly elastic in texture, and is less likely to be blocked in flow channels than hard materials do.

7. The double layer microsphere according to claim 1, **characterized in that** the microsphere can independently occupy a flow channel and being arranged in order, so that time intervals of the microspheres entering the subsequent flow channel are constant, which is beneficial for controlling a speed of the microspheres entering the main flow channel using flow rate and is also beneficial for achieving high singlet rate of microsphere encapsulation in droplets.

8. A method for preparing a double layer microsphere with oligonucleotide sequences according to claim 4, including:
(1) synthesizing, respectively, three oligonucleotide sequences C+D1, D2 and D3, and corresponding auxiliary sequences RC2 and RC3 wherein, RC2 is a complementary sequence of ACTG [Y] ACTG, and RC 3 is a complementary sequence of [Z] [polyT];
(2) adding the oligonucleotide sequence C+D1 into a microporous plate, and adding the magnetic microspheres to connect with C+D1 to obtain a magnetic microsphere mixture A;
(3) adding DNA ligase into the magnetic microsphere mixture A obtained in step (2) to obtain a mixed system M; adding the oligonucleotide sequence D2 into a new microporous plate, adding the auxiliary sequence RC2 to obtain a microporous plate containing D2; adding the mixed system M into the microporous plate containing D2 to obtain a polyacrylamide microsphere mixture B connected with D1 and D2;
(4) adding DNA ligase into the magnetic microsphere mixture B obtained in step (3) to obtain a mixed system N, adding the oligonucleotide sequence D3 into a new microporous plate, adding the auxiliary sequence RC3 to obtain a microporous plate containing D3; adding the mixed system N into the microporous plate containing D3 to obtain the magnetic microsphere connected with D1, D2 and D3.
(5) by means of droplet microfluidic method, coating the magnetic microsphere having molecular barcodes sequence generated in step (4) into a polyacrylamide material to form a double layer microsphere.

## Patentansprüche

1. Doppelschicht-Mikrokügelchen mit Oligonukleotidsequenzen, **dadurch gekennzeichnet, dass** eine Außenschicht des Doppelschicht-Mikrokügelchens aus einem löslichen Hydrogel hergestellt ist und die Innenschicht davon ein magnetisches Mikrokügelchen mit Oligonukleotidsequenzen ist;
die Oligonukleotidsequenz mindestens zwei Teile, eine C-Region und eine D-Region, aufweist; die C-Region eine universelle DNA-Sequenz zum Bilden einer Sequenzierungsbibliothek ist; und die Länge der D-Region-Sequenz 10 bp bis 200 bp beträgt und zum Identifizieren von Nukleinsäuren in unterschiedlichen einzelnen Zellen verwendet wird und eine Sequenz zum Kombinieren und Einfangen von Nukleinsäure in Zellen beinhaltet.

2. Doppelschicht-Mikrokügelchen nach Anspruch 1, **dadurch gekennzeichnet, dass** die Oligonukleotidsequenzen durch chemische Bindungen, die mithilfe eines Verfahrens der enzymatischen Verbindung hergestellt wurden, in Sequenz mit den magnetischen Mikrokügelchen der Innenschicht verbunden sind.

3. Doppelschicht-Mikrokügelchen nach Anspruch 1, **dadurch gekennzeichnet, dass** der Durchmesser der Außenschicht des Mikrokügelchens 30 bis 300 Mikrometer beträgt.

4. Doppelschicht-Mikrokügelchen nach Anspruch 1, **dadurch gekennzeichnet, dass** die D-Region-Sequenz eines inneren magnetischen Mikrokügelchens mindestens drei Teile, D1, D2 und D3, beinhaltet, wobei die Sequenz von D1 ACACTCTTTCCCTACACGACGCTCTTCCGATCT [X] A ist, die Sequenz von D2 CTG [Y] A ist und die Sequenz von D3 CTG [Z] [polyT] ist; wobei X, Y, Z jeweils eine DNA-Sequenz mit einer Länge von 4 bis 14 bp darstellen und polyT 10 bis 35 T darstellt.

5. Doppelschicht-Mikrokügelchen nach Anspruch 1, **dadurch gekennzeichnet, dass** die Außenschicht aus Disulfidbindungen enthaltendem Polyacrylamid hergestellt ist.

6. Doppelschicht-Mikrokügelchen nach Anspruch 1, **dadurch gekennzeichnet, dass** eine Struktur des Mikrokügelchens eine multi-mikroporöse massive Kügelchenstruktur ist und eine weiche, zähe und leicht elastische Textur aufweist und in Strömungskanälen weniger wahrscheinlich als harte Materialien blockiert wird.

7. Doppelschicht-Mikrokügelchen nach Anspruch 1, **dadurch gekennzeichnet, dass** das Mikrokügelchen unabhängig einen Strömungskanal belegen kann und in Reihenfolge angeordnet ist, so dass Zeitintervalle der Mikrokügelchen, die in den nachfolgenden Strömungskanal eintreten, konstant sind, was zum Steuern einer Geschwindigkeit der Mikrokügelchen vorteilhaft ist, die in den Hauptströmungskanal eintreten, und auch vorteilhaft ist, was das Erzielen einer hohen Singulettrate einer Mikrokügelchenverkapselung in Tropfen betrifft.

8. Verfahren zum Herstellen eines Doppelschicht-Mikrokügelchens mit Oligonukleotidsequenzen nach Anspruch 4, das beinhaltet:
(1) jeweiliges Synthetisieren von drei Oligonukleotidsequenzen C+D1, D2 und D3 und entsprechender Hilfssequenzen RC2 und RC3, wobei RC2 eine komplementäre Sequenz von ACTG [Y] ACTG ist und RC 3 eine komplementäre Sequenz von [Z] [polyT] ist;
(2) Zusetzen der Oligonukleotidsequenz C+D1 in eine mikroporöse Platte und Zusetzen der magnetischen Mikrokügelchen zur Verbindung mit C+D1, um eine magnetisches Mikrokügelchengemisch A zu erhalten;
(3) Zusetzen von DNA-Ligase in das magnetische Mikrokügelchengemisch A, das in Schritt (2) erhalten wurde, um ein gemischtes System M zu erhalten; Zusetzen der Oligonukleotidsequenz D2 in eine neue mikroporöse Platte, Zusetzen der Hilfssequenz RC2, um eine D2-haltige mikroporöse Platte zu erhalten; Zusetzen des gemischten Systems M in die D2-haltige mikroporöse Platte, um ein Polyacrylamid-Mikrokügelchengemisch B zu erhalten, das mit D1 und D2 verbunden ist;
(4) Zusetzen von DNA-Ligase in das magnetische Mikrokügelchengemisch B, das in Schritt (3) erhalten wurde, um ein gemischtes System N zu erhalten; Zusetzen der Oligonukleotidsequenz D3 in eine neue mikroporöse Platte, Zusetzen der Hilfssequenz RC3, um eine D3-haltige mikroporöse Platte zu erhalten; Zusetzen des gemischten Systems N in die D3-haltige mikroporöse Platte, um das magnetische Mikrokügelchen zu erhalten, das mit D1, D2 und D3, verbunden ist.
(5) mithilfe eines Verfahrens der tropfenbasierten Mikrofluidik Beschichten des magnetischen Mikrokügelchens mit molekularer Strichcodesequenz, wie in Schritt (4) erzeugt, in ein Polyacrylamidmaterial, um ein Doppelschicht-Mikrokügelchen zu bilden.

## Revendications

1. Microsphère à double couche avec des séquences oligonucléotidiques, **caractérisée en ce qu'**une couche externe de la microsphère à double couche est constituée d'un hydrogel soluble, et la couche interne de celle-ci est une microsphère magnétique avec des séquences oligonucléotidiques ;
la séquence oligonucléotidique possède au moins deux parties, une région C et une région D ; la région C est une séquence d'ADN universelle pour la construction d'une bibliothèque de séquençage ; et la longueur de la séquence de la région D est de 10 pb-200 pb, et est utilisée pour identifier des acides nucléiques dans différentes cellules individuelles, et comprend une séquence pour combiner et capturer un acide nucléique dans les cellules.

2. Microsphère à double couche selon la revendication 1, **caractérisée en ce que** les séquences oligonucléotidiques sont connectées aux microsphères magnétiques de la couche interne en séquence par l'intermédiaire de liaisons chimiques créées par un procédé de connexion enzymatique.

3. Microsphère à double couche selon la revendication 1, **caractérisée en ce que** le diamètre de la couche externe de la microsphère est de 30-300 microns.

4. Microsphère à double couche selon la revendication 1, **caractérisée en ce que** la séquence de la région D d'une microsphère magnétique interne comprend au moins trois parties, D1, D2 et D3, dans laquelle la séquence de D1 est ACACTCTTTCCCTACACGACGCTCTTCCGATCT [X] A, la séquence de D2 est CTG [Y] A, et la séquence de D3 est CTG [Z] [polyT] ; dans laquelle X, Y, Z représentent chacun une séquence d'ADN possédant une longueur de 4-14 pb respectivement, et polyT représente 10-35 T.

5. Microsphère à double couche selon la revendication 1, **caractérisée en ce que** la couche externe est constituée de polyacrylamide contenant des liaisons disulfure.

6. Microsphère à double couche selon la revendication 1, **caractérisée en ce qu'**une structure de la microsphère est une structure de sphère solide multi-microporeuse, et sa texture est souple, résistante et légèrement élastique, et est moins susceptible d'être bloquée dans des canaux d'écoulement que des matériaux durs.

7. Microsphère à double couche selon la revendication 1, **caractérisée en ce que** la microsphère peut indépendamment occuper un canal d'écoulement et est arrangée dans l'ordre, de sorte que les intervalles de temps des microsphères entrant dans le canal d'écoulement suivant sont constants, ce qui est bénéfique pour contrôler la vitesse des microsphères entrant dans le canal d'écoulement principal en utilisant le débit, et est également bénéfique pour obtenir un taux de singulet élevé en termes d'encapsulation des microsphères dans des gouttelettes.

8. Procédé de préparation d'une microsphère à double couche avec des séquences oligonucléotidiques selon la revendication 4, comprenant les étapes consistant à :
(1) synthétiser, respectivement, trois séquences oligonucléotidiques C+D1, D2 et D3, et des séquences auxiliaires correspondantes RC2 et RC3 dans lesquelles RC2 est une séquence complémentaire de ACTG [Y] ACTG, et RC3 est une séquence complémentaire de [Z] [polyT] ;
(2) ajouter la séquence oligonucléotidique C+D1 dans une plaque microporeuse, et ajouter les microsphères magnétiques pour les connecter avec C+D1 afin d'obtenir un mélange de microsphères magnétiques A ;
(3) ajouter une ADN ligase dans le mélange de microsphères magnétiques A obtenu à l'étape (2) pour obtenir un système mixte M ; ajouter la séquence oligonucléotidique D2 dans une nouvelle plaque microporeuse, ajouter la séquence auxiliaire RC2 afin d'obtenir une plaque microporeuse contenant D2 ; ajouter le système mixte M dans la plaque microporeuse contenant D2 afin d'obtenir un mélange de microsphères en polyacrylamide B connectées avec D1 et D2 ;
(4) ajouter une ADN ligase dans le mélange de microsphères magnétiques B obtenu à l'étape (3) pour obtenir un système mixte N ; ajouter la séquence oligonucléotidique D3 dans une nouvelle plaque microporeuse, ajouter la séquence auxiliaire RC3 afin d'obtenir une plaque microporeuse contenant D3 ; ajouter le système mixte N dans la plaque microporeuse contenant D3 afin d'obtenir la microsphère magnétique connectée avec D1, D2 et D3 ;
(5) à l'aide d'un procédé microfluidique à base de gouttelettes, revêtir la microsphère magnétique comportant une séquence à codes-barres moléculaires générée à l'étape (4) dans un matériau en polyacrylamide afin de former une microsphère à double couche.
